(19) Europäisches Patentamt / European Patent Office / Office européen des brevets

(11) **EP 2 249 898 B1**

(12) **EUROPÄISCHE PATENTSCHRIFT**

(45) Veröffentlichungstag und Bekanntmachung des Hinweises auf die Patenterteilung:
**01.05.2019 Patentblatt 2019/18**

(21) Anmeldenummer: **09700331.3**

(22) Anmeldetag: **08.01.2009**

(51) Int Cl.:
***A61M 1/34*** (2006.01)

(86) Internationale Anmeldenummer:
**PCT/EP2009/000054**

(87) Internationale Veröffentlichungsnummer:
**WO 2009/087092 (16.07.2009 Gazette 2009/29)**

(54) **VERFAHREN ZUM BESTIMMEN DES ANTEILS DER REZIRKULATION IN EINER FISTEL UND/ODER DER KARDIOPULMONALEN REZIRKULATION AN DER SUMME VON FISTELREZIRKULATION UND KARDIOPULMONALER REZIRKULATION**

METHOD FOR DETERMINING THE PERCENTAGE OF RECIRCULATION IN A FISTULA AND/OR CARDIOPULMONARY RECIRCULATION RELATIVE TO THE TOTAL FISTULA RECIRCULATION AND CARDIOPULMONARY RECIRCULATION

PROCÉDÉ DE DÉTERMINATION DE LA FRACTION DE RECIRCULATION DANS UNE FISTULE ET/OU DE LA RECIRCULATION CARDIOPULMONAIRE, PAR RAPPORT À LA SOMME DE LA RECIRCULATION DANS LA FISTULE ET DE LA RECIRCULATION CARDIOPULMONAIRE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **09.01.2008 DE 102008003714**

(43) Veröffentlichungstag der Anmeldung:
**17.11.2010 Patentblatt 2010/46**

(73) Patentinhaber: Fresenius Medical Care
**Deutschland GmbH**
**61342 Bad Homburg v.d.H. (DE)**

(72) Erfinder: **ZHANG, Wei**
**97464 Niederwerrn (DE)**

(74) Vertreter: **Oppermann, Frank et al**
**OANDO Oppermann & Oppermann LLP**
**Wilhelminenstrasse 1a**
**65193 Wiesbaden (DE)**

(56) Entgegenhaltungen:
**EP-A- 0 189 561    WO-A-01/45770**
**WO-A-2006/072271**

**Beschreibung**

Die Erfindung bezieht sich auf eine Vorrichtung zum Bestimmen des Anteils der Rezirkulation in einer Fistel und/oder der kardiopulmonalen Rezirkulation an der Summe von Fistelrezirkulation und kardiopulmonaler Rezirkulation für eine Vorrichtung zur extrakorporalen Blutbehandlung mit einem extrakorporalen Blutkreislauf, der eine erste Kammer eines durch eine Membran in die erste Kammer und eine zweite Kammer unterteilten Dialysators oder Filters einschließt, und einem Flüssigkeitssystem, dass die zweite Kammer des Dialysators oder Filters einschließt, wobei der ersten Kammer des Dialysators oder Filters Blut mit einer bestimmten Blutflussrate zugeführt wird und dem Blut stromauf oder stromab der ersten Kammer des Dialysators oder Filters Substitutionsflüssigkeit mit einer bestimmten Substitutionsrate zugeführt wird und dem Blut über die Membran des Dialysators oder Filters mit einer bestimmten Flussrate Flüssigkeit entzogen wird.

Darüber hinaus betrifft die Erfindung eine extrakorporale Blutbehandlungsvorrichtung mit einer Vorrichtung zur Bestimmung des Anteils der Fistelrezirkulation und/oder der kardiopulmonalen Rezirkulation an der Summe von Fistel- und kardiopulmonaler Rezirkulation.

Zur Entfernung von harnpflichtigen Substanzen und zum Flüssigkeitsentzug werden verschiedene Verfahren zur extrakorporalen Blutbehandlung bzw, -reinigung eingesetzt. Bei der Hämodialyse wird das Blut des Patienten außerhalb des Körpers in einem Dialysator gereinigt. Der Dialysator weist eine Blutkammer und eine Dialysierflüssigkeitskammer auf, die durch eine semipermeable Membran getrennt sind. Während der Behandlung strömt Blut des Patienten durch die Blutkammer, Um das Blut effektiv von harnpflichtigen Substanzen zu reinigen, wird die Dialysierflüssigkeitskammer kontinuierlich von frischer Dialysierflüssigkeit durchströmt.

Während bei der Hämodialyse (HD) der Transport der kleineren molekularen Substanzen durch die Membran des Dialysators im Wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe, durch einen hohen Flüssigkeitsstrom (Konvektion) durch die Membran des Dialysators effektiv entfernt. Bei der Hämofiltration fungiert der Dialysator als Filter. Eine Kombination aus beiden Verfahren ist die Hämodialfiltration (HDF).

Bei der Hämo(dia)filtration wird ein Teil der dem Blut durch die Membran des Dialysators entzogenen Flüssigkeit durch eine sterile Substitutionsflüssigkeit ersetzt, die im Allgemeinen entweder stromauf des Dialysators oder stromab des Dialysators dem extrakorporalen Blutkreislauf zugeführt wird. Die Zufuhr der Substitutionsflüssigkeit stromauf des Dialysators wird auch als Prädilution und die Zufuhr stromab des Dialysators als Postdilution bezeichnet.

Es sind Vorrichtungen zur Hämo(dia)filtration bekannt, bei denen die Dialysierflüssigkeit online aus Frischwasser und Dialysierflüssigkeitskonzentrat und die Substitutionsflüssigkeit online aus der Dialysierflüssigkeit hergestellt werden.

Bei den bekannten Hämo(dia)filtrationsvorrichtungen wird die Substitutionsflüssigkeit (Substituat) dem extrakorporalen Blutkreislauf von dem Flüssigkeitssystem der Maschine über eine Substituatzuführleitung zugeführt. Bei der Prädilution fuhrt die Substituatleitung zu einer Anschlussstelle an der arteriellen Blutleitung stromauf des Dialysators oder Filters, während bei der Postdilution die Substituatleitung zu einer Anschlussstelle an der venösen Blutleitung stromab des Dialysators oder Filters führt. Die Substituatleitung weist beispielsweise einen Konnektor auf, mit dem sie entweder an die venöse oder arterielle Blutleitung angeschlossen werden kann. Zum Unterbrechen der Flüssigkeitszufuhr ist an der Substituatleitung eine Klemme oder dgl. vorgesehen. Ein derartiges Hämo(dia)filtrationsgerät ist beispielsweise aus der EP-A-0 189 561 bekannt.

Bei den bekannten Verfahren der chronischen Blutreinigungstherapie, wie Hämodialyse, Hämofiltration und Hämodiafiltration, wird als Zugang zum Blutgefäßsystem des Patienten häufig operativ eine arteriovenöse Fistel angelegt. Ebenso ist der Einsatz eines Implantats möglich. Wenn nachfolgend von einer "Fistel" die Rede ist, wird darunter jede Art der Verbindung zwischen einer Vene und einer Arterie des Patienten verstanden.

Das durch die Fistel fließende Blut wird nur während der eigentlichen Dialysebehandlung benutzt. Im dialysefreien Zeitraum entspricht der Blutfluss in der Fistel einem funktionellen Links/Rechts-Shunt, bei dem ein Anteil des arteriellen Blutes aus dem Herzminutenvolumen (HMV) unter Umgehung einer peripheren Nutzung direkt dem venösen System und dem Herzen zugeführt wird. Der Fistelfluss rezirkuliert über Herz und Lungen. Der fraktionelle Anteil des Fistelflusses am Herzminutenvolumen wird als kardiopulmonare Rezirkulation definiert.

Die kardiopulmonare Rezirkulation hat nicht nur Auswirkungen auf die Kreislaufbelastung des Patienten, sondern auch auf die Effizienz der Dialyse. Da das dialysierte Blut aus dem extrakorporalen Kreislauf unter Umgehung der systemischen Kreislaufgebiete dem venösen Rückstrom aus dem großen Körperkreislauf beigemischt wird, kommt es zu einer systematischen Reduktion in der Konzentration dialysierbarer Bestandteile im arteriellen Blut (D. Schneditz et al.: Cardiopulmonary recirculation during hemodialysis. Kidney Int. 42: 1450 -1456, 1992).

Für die Funktionsfähigkeit der Fistel ist deren Perfusion von Bedeutung. Sinkt der Fistelfluss unter einen kritischen Wert, dann steigt das Risiko einer Fistelthrombose mit dem möglichen Verlust des Gefäßzuganges, was in der Dialysebehandlung eine erhebliche Komplikation darstellt (W. Bay et al.: Color Doppler flow predicts PTFE graft failure, J. Am. Soc, Nephrol. 5: 407 (1994)). Ist der Fistelfluss während der Dialysebehandlung kleiner als der extrakorporale Blutfluss ($Q_B$), kommt es zur lokalen Fistelrezirkulation, wobei eine Fraktion des dialysierten und mit der venösen Blutleitung zur Fistel zurückgeführten Bluts über die arterielle Blutleitung dem Dialysator wieder zugeführt wird. Die Fistelrezirkulation $R_A$ bewirkt eine bedeutende Verminderung der Dialyseeffizienz (F. Gotch: "Models to predict recirculation and its effect on treatment time in single-needle dialysis" First Intl. Symposium on Single-Needle Dialysis, Hrsg.: S. Rignoir. R. Vanholder und P. Invanovich, Cleveland, ISAO Press, 1984, Seite 305 ff.). Die Messung der Qualität des Gefäßzuganges ist somit ein wichtiges Mittel zur Qualitätssicherung bei der Dialysebehandlung.

Aufgrund ihrer klinischen Bedeutung sind verschiedene Verfahren zur Messung der Fistelrezirkulation ($R_A$) bekannt. Allen gemeinsam ist die Messung einer physikalischen oder chemischen Kenngröße des Blutes, die im venösen Zweig des extrakorporalen Kreislaufs verändert wird. Die physikalische oder chemische Kenngröße des Blutes kann durch eine manuelle Injektion eines Indikators oder auch mittelbar über die Dialyseaufbereitungseinheit geändert werden.

Aus EDTNA-ERCA Journal 19, 6 (1993) ist ein als Thermodilution bezeichnetes Verfahren zur Rezirkulationsmessung bekannt. Bei dem bekannten Verfahren wird ein kurzzeitiger Temperaturabfall im Dialysierflüssigkeitskreislauf iniziert, der sich auf den venösen Zweig des extrakorporalen Kreislaufs überträgt und zu einem nachweisbaren Temperatursprung im arteriellen Zweig des extrakorporalen Kreislaufs dann führt, wenn eine Rezirkulation auftritt.

Eine bekannte Vorrichtung zur Durchführung des als Thermodilution bezeichneten Verfahrens weist einen im arteriellen Zweig und einen im venösen Zweig des extrakorporalen Kreislaufs angeordneten Temperaturfühler auf. Mit dem venösen Temperaturfühler wird der Temperatursprung aufgenommen, der auf den im Dialysierflüssigkeitskreislauf erzeugten Temperaturabfall zurückzuführen ist. Der gemessene Temperatursprung wird zeitlich integriert oder anders charakterisiert und nachfolgend mit dem im arteriellen Messfühler registrierten Temperaturverlauf verglichen. Das Verhältnis der beiden Temperatur-Integrale oder anderer Kenngrößen zueinander ist ein Maß für die gesamte Effizienzminderung der Dialysebehandlung durch fistel- und kardiopulmonale Rezirkulation.

Die bekannte Vorrichtung zur Rezirkulationsmessung hat sich in der Praxis bewährt. Als nachteilig erweist sich jedoch, dass nur die nachfolgend als Rezirkulation R bezeichnete Gesamtrezirkulation gemessen werden kann, die der Summe der Fistelrezirkulation $R_A$ und der kardiopulmonalen Rezirkulation $R_{CP}$ entspricht.

Ein Verfahren zur Messung der Rezirkulation R, d.h. der Summe der Fistelrezirkulation ($R_A$) und der kardiopulmonalen Rezirkulation $R_{CP}$, ist auch aus der DE 197 02 441 C1 bekannt. Bei dem bekannten Verfahren wird eine physikalische oder chemische Kenngröße der Dialysierflüssigkeit im Dialysierflüssigkeitsweg stromauf des Dialysators verändert, die zu einer Änderung der physikalischen oder chemischen Kenngröße auf der Blutseite führt. Die Änderung der Kenngröße der Dialysierflüssigkeit auf der Blutseite führt zu einer Änderung der Kenngröße der Dialysierflüssigkeit stromab der Dialysierflüssigkeitskammer des Dialysators. Zur Bestimmung der Rezirkulation wird die Kenngröße im Dialysierflüssigkeitsweg stromab des Dialysators gemessen und aus dem zeitlichen Verlauf der Änderung der Kenngröße wird die Rezirkulation R bestimmt. Als physikalische oder chemische Kenngröße kann die Dialysierflüssigkeits-Ionen-Konzentration, beispielsweise die Na-Konzentration der Dialysierflüssigkeit, oder auch die Temperatur der Dialysierflüssigkeit verändert und gemessen werden. Nachteilig ist jedoch wieder, dass mit dem bekannten Verfahren nicht zwischen der Fistelrezirkulation $R_A$ und der kardiopulmonalen Rezirkulation $R_{CP}$ unterschieden werden kann.

Die DE-A-195 28 907 CI beschreibt ein Verfahren zur Bestimmung der kardiopulmonalen Rezirkulation. Die Messung der kardiopulmonalen Rezirkulation beruht auf zwei kurz aufeinander folgenden Messungen der Rezirkulationsfraktion, die automatisch vor und nach der Umkehrung des Blutflusses durchgeführt werden. Nachteilig ist, dass das bekannte Verfahren die Umkehr des Blutflusses erfordert.

Aus der US 6 537 240 B2 ist ein Verfahren zur Bestimmung der Rezirkulation bekannt, das auf einer Änderung der Zusammensetzung des Bluts im extrakorporalen Blutkreislauf auf Grund einer Erhöhung oder Verringerung der Ultrafiltrationsrate innerhalb eines vorgegebenen Zeitintervalls beruht.

Aus der WO 01/45770 A1 ist eine Vorrichtung zur Bestimmung der Rezirkulation bekannt, die auf einer Veränderung der Hämoglobinkonzentration in der venösen Blutleitung und der Erfassung der Veränderung der Hämoglobinkonzentration in der arteriellen Blutleitung beruht.

Die WO 2006/072271 A1 beschreibt eine Vorrichtung zur Erkennung von Komplikationen während einer extrakorporalen Blutbehandlung, die auf der Kombination der Überwachung der Dialysance auf der Grundlage von dialysatseitigen Messgrößen und der Überwachung der Rezirkulation auf der Grundlage von blutseitigen Messgrößen beruht.

Der Erfindung liegt die Aufgabe zu Grunde, eine Vorrichtung zur Bestimmung des Anteils der Fistelrezirkulation und/oder der kardiopulmonalen Rezirkulation an der Summe von Fistelrezirkulation und kardiopulmonaler Rezirkulation bereitzustellen. Eine weitere Aufgabe der Erfindung ist, eine extrakorporale Blutbehandlungsvorrichtung zu schaffen, die über eine Vorrichtung zur Bestimmung des Anteils der Fistelrezirkulation und/oder der kardiopulmonalen Rezirkulation an der Summe von Fistel- und kardiopulmonaler Rezirkulation verfügt.

Die Lösung dieser Aufgaben erfolgt erfindungsgemäß mit den Merkmalen der unabhängigen Patentansprüche. Vorteilhafte Ausführungsformen der Erfindung sind Gegenstand der Unteransprüche.

Die erfindungsgemäße Vorrichtung zum Bestimmen des Anteils der Fistelrezirkulation und/oder kardiopulmonalen Rezirkulation an der Summe von Fistel- und kardiopulmonaler Rezirkulation, die nachfolgend auch als Rezirkulation bezeichnet wird, setzt eine extrakorporale Blutbehandlung voraus, bei der dem Blut stromauf oder stromab der ersten Kammer des Dialysators oder Filters Substitutionsflüssigkeit mit einer bestimmten Substitutionsrate zugeführt wird und dem Blut über die Membran des Dialysators oder Filters mit einer bestimmten Flussrate Flüssigkeit entzogen wird. Die erfindungsgemäße Vorrichtung beruht darauf, dass stromauf oder stromab des Dialysators oder Filters die Substitutionsrate für ein vorgegebenes Zeitintervall um einen vorgegebenen Betrag verändert wird, während die Flussrate der durch die Membran des Dialysators entzogenen Flüssigkeit verändert wird, so dass ein zeitlich begrenzter Bolus erzeugt wird.

Vor der Veränderung der Substitutionsrate oder der Flussrate der durch die Membran des Dialysators oder Filters entzogenen Flüssigkeit wird das Blutvolumen $RBV(t_1)$ oder eine mit dem Blutvolumen korrelierende Größe, beispielsweise der Hämatokrit $Hct(t_1)$, die Blutdichte oder Hämoglobin-Konzentration bestimmt. Wenn das Blutvolumen oder der Hämatokrit vor der Veränderung der Substitutionsrate oder der Flussrate der durch die Membran entzogenen Flüssigkeit bekannt ist, erübrigt sich die Messung des Blutvolumens oder des Hämatokrits.

Nach der Veränderung der Substitutionsrate oder der Flussrate der durch die Membran entzogenen Flüssigkeit wird wieder das Blutvolumen oder die mit dem Blutvolumen korrelierende Größe bestimmt, um die Änderung des Blutvolumens oder der mit dem Blutvolumen korrelierenden Größe infolge der Veränderung der Substitutionsrate oder der Flussrate der durch die Membran entzogenen Flüssigkeit erfassen zu können. Grundsätzlich ist es nicht erforderlich, das Blutvolumen vor und nach der Veränderung der Flussraten als absolute Größen zu bestimmen, sondern es genügt, die Bestimmung der Veränderung des Blutvolumens infolge der Veränderung der Substitutionsrate oder der Flussrate der durch die Membran des Dialysators entzogenen Flüssigkeit. Beispielsweise ist es ausreichend, den Quotienten aus dem relativen Blutvolumen vor und nach der Veränderung der Flussraten zu bestimmen.

Die Fistelrezirkulation $R_A$ wird auf der Grundlage des Vergleichs des Blutvolumens oder der mit dem Blutvolumen korrelierenden Größe vor und nach der Veränderung der Substitutionsrate und der Flussrate der durch die Membran entzogenen Flüssigkeit bestimmt. Die Bestimmung der Fistelrezirkulation $R_A$ ist also allein auf Grund der Änderung des Blutvolumens möglich. Da die geänderten Blutvolumenwerte relativ schnell erreicht werden, zeichnet sich die Messung der Fistelrezirkulation durch eine relativ kurze Messzeit aus, die in der Praxis zwischen 1 und 2 Minuten liegen kann. Die relativ kurze Messzeit hat zur Folge, dass nur der Anteil der Fistelrezirkulation, nicht aber der Anteil der kardiopulmonalen Rezirkulation an der Rezirkulation R, d.h. der Summe von Fistelrezirkulation und kardiopulmonaler Rezirkulation, erfasst wird. Da die kardiopulmonale Rezirkulation erst nach Ablauf der relativ kurzen Messzeit auftritt, wird dieser Anteil an der Rezirkulation nicht erfasst.

Zur Bestimmung des Anteils der Fistelrezirkulation $R_A$ an der Rezirkulation R wird die Rezirkulation R nach den bekannten Verfahren ermittelt. Die Rezirkulation R kann beispielsweise nach dem als Thermodilution bekannten Verfahren bestimmt werden (EDTNA-ERCA Journal 19,6 (1993)). Es ist aber auch möglich, die Rezirkulation nach anderen bekannten Verfahren zu bestimmen.

Sobald die Rezirkulation R bestimmt ist, wird der Anteil der Fistelrezirkulation $R_A$ an der Rezirkulation R und/oder der kardiopulmonalen Rezirkulation $R_{CP}$ an der Rezirkulation R bestimmt. Die kardiopulmonale Rezirkulation $R_{CP}$ kann nach der Bestimmung der Fistelrezirkulation $R_A$ aus der Differenz der Rezirkulation R und der Fistelrezirkulation $R_A$ berechnet werden.

Die erfindungsgemäße Vorrichtung zeichnet sich also im Wesentlichen durch die Kombination einer schnellen Bestimmung der Fistelrezirkulation auf der Grundlage einer Veränderung der Substitutionsrate und der Flussrate der durch die Membran des Dialysators oder Filters entzogenen Flüssigkeit und der Bestimmung der Summe von Fistelrezirkulation und kardiopulmonaler Rezirkulation aus, um die jeweiligen Anteile von Fistel- und kardiopulmonaler Rezirkulation an der Rezirkulation bestimmen zu können.

Die Bestimmung der Fistelrezirkulation auf der Grundlage der Veränderung der Substitutionsrate und der Flussrate der durch die Membran entzogenen Flüssigkeit ist darauf zurückzuführen, dass die Veränderung dieser Flussraten eine Veränderung der Dichte des Blutes oder die Konzentration eines Blutinhaltsstoffes zur Folge hat. Es bildet sich im extrakorporalen Blutkreislauf eine Art verdickte oder verdünnte "Blutsäule" aus, die einen zeitlich begrenzten "Bolus" darstellt, der so schnell in der Fistel rezirkuliert, dass er im extrakorporalen Blutkreislauf nachgewiesen werden kann, bevor die langsamere kardiopulmonare Rezirkulation einsetzt. Folglich wird mit der relativ schnellen Messung die Fistelrezirkulation, nicht aber die kardiopulmonale Rezirkulation nachgewiesen, so dass nach der Messung der Summe von Fistel- und kardiopulmonarer Rezirkulation mit den bekannten Messverfahren die jeweiligen Anteile von Fistel- und/oder kardiopulmonaler Rezirkulation an der Rezirkulation bestimmt werden können.

Grundsätzlich ist es unerheblich, ob das Blutvolumen oder die mit dem Blutvolumen korrelierende Größe, beispielsweise der Hämatokrit im arteriellen oder venösen Zweig des extrakorporalen Blutkreislaufs bestimmt wird, da nach einer Messung dieser charakteristischen Größe im arteriellen bzw. venösen Zweig die Größe im venösen bzw. arteriellen Zweig des extrakorporalen Kreislaufs berechnet werden kann.

Eine bevorzugte Ausführungsform der erfindungsgemäßen Vorrichtung sieht eine Verringerung der Substitutionsrate und der Flussrate der durch die Membran des Dialysators oder Filters entzogenen Flüssigkeit um den gleichen Betrag vor.

Bei einer weiteren Ausführungsform wird die Zufuhr von Substitutionsflüssigkeit stromauf oder stromab des Dialysators oder Filters und der Entzug von Flüssigkeit über die Membran des Dialysators oder Filters vollständig unterbrochen. Die Unterbrechung erfolgt vorzugsweise für ein vorgegebenes Zeitintervall, so dass sich nach Ablauf des Zeitintervalls wieder die ursprünglichen Verhältnisse einstellen.

Es ist aber grundsätzlich auch möglich, dass die Substitutionsrate und die Flussrate der durch die Membran des Dialysators oder Filters entzogenen Flüssigkeit, vorzugsweise um den gleichen Betrag, vergrößert wird oder eine Zufuhr von Substitutionsflüssigkeit stromauf oder stromab des Dialysators oder Filters und ein Entzug von Flüssigkeit über die Membran des Dialysators oder Filters vorgenommen wird, wobei das Blutvolumen oder die mit dem Blutvolumen korrelierende Größe vorher und nachher gemessen wird. Auch dabei kommt es wieder zu der Ausbildung einer "Blutsäule", wobei sich das Blut bei der Postdilution verdünnt und bei der Prädilution verdickt.

Die Implementierung des erfindungsgemäßen Verfahrens in die bekannten Blutbehandlungsvorrichtungen erfordert keinen großen apparativen Aufwand, da es lediglich erforderlich ist, die Substitutionspumpe kurzzeitig zu stoppen und den Dialysator oder Filter von dem extrakorporalen Blutkreislauf zu trennen, so dass Flüssigkeit nicht über die Membran des Dialysators oder Filters entzogen werden kann. Beispielsweise kann die Messung zusammen mit den bekannten Druckhaltetests bei Blutbehandlungsvorrichtungen durchgeführt werden, bei denen die Substitutionspumpe angehalten und der Dialysator in einen Bypass geschaltet wird.

Im folgenden wird ein Ausführungsbeispiel einer erfindungsgemäßen Blutbehandlungsvorrichtung, die über eine erfindungsgemäße Vorrichtung zum Bestimmen des Anteils der Fistelrezirkulation und/oder kardiopulmonalen Rezirkulation an der Summe von Fistel- und kardiopulmonaler Rezirkulation verfügt, unter Bezugnahme auf die Zeichnungen im Einzelnen beschrieben.

Es zeigen:

Fig. 1      eine extrakorporale Blutbehandlungsvorrichtung mit einer Vorrichtung zum Bestimmen des Anteils der Fistelrezirkulation und/oder der kardiopulmonalen Rezirkulation an der Summe von Fistel- und kardiopulmonaler Rezirkulation in stark vereinfachter schematischer Darstellung,

Fig. 2      das relative Blutvolumen als Funktion der Zeit während einer extrakorporalen Blutbehandlung und

Fig. 3      eine vergrößerte Darstellung des zeitlichen Verlaufs des relativen Blutvolumens während eines Abschnitts der extrakorporalen Blutbehandlung.

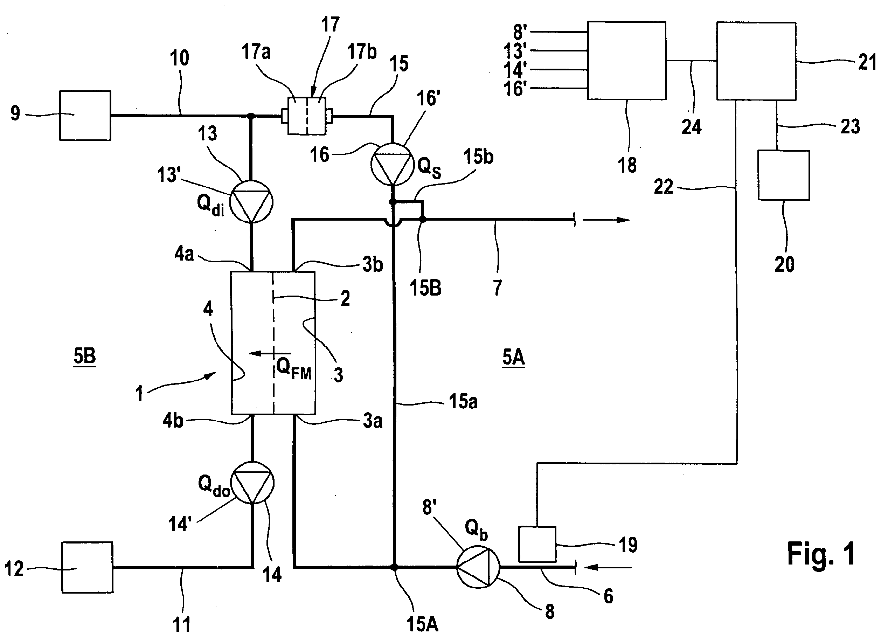

Fig. 1 zeigt nur die wesentlichen Komponenten der extrakorporalen Blutbehandlungsvorrichtung in schematischer Darstellung, die für die Bestimmung des Anteils der Fistelrezirkulation und/oder der kardiopulmonalen Rezirkulation an der Rezirkulation relevant sind.

Bei der vorliegenden Blutbehandlungsvorrichtung handelt es sich um eine Hämo(dia)filtrationsvorrichtung, die einen Dialysator 1 aufweist, der durch eine semipermeable Membran 2 in eine von Blut durchflossene erste Kammer 3, die nachfolgend als Blutkammer bezeichnet wird und eine von Dialysierflüssigkeit durchflossene zweite Kammer 4 getrennt ist, die als Dialysierflüssigkeitskammer bezeichnet wird. Die erste Kammer 3 ist in einen extrakorporalen Blutkreislauf 5A geschaltet, während die zweite Kammer 4 in das Dialysierflüssigkeitssystem 5B der Hämo(dia)filtrationsvorrichtung geschaltet ist.

Der extrakorporale Blutkreislauf 5A umfasst eine arterielle Blutleitung 6, die zu dem Einlass 3a der Blutkammer 3 führt, und eine venöse Blutleitung 7, die von dem Auslass 3b der Blutkammer 3 des Dialysators 1 abgeht. Das Blut des Patienten wird durch die Blutkammer 3 des Dialysators 1 mit einer arteriellen Blutpumpe 8, insbesondere Rollenpumpe gefördert, die an der arteriellen Blutleitung 6 angeordnet ist. Die Blutpumpe führt der Blutkammer 3 des Dialysators Blut mit einer bestimmten Blutflussrate $Q_b$ zu. Die Blutleitungen 6, 7 und der Dialysator 3 bilden ein zur einmaligen Verwendung bestimmtes Disposable, das für die Dialysebehandlung in die Dialysevorrichtung eingelegt wird. Zur Eliminierung von Luftblasen können in die arterielle und venöse Blutleitung Luftabscheider (Tropfkammer) geschaltet sein.

Die frische Dialysierflüssigkeit wird in einer Dialysierflüssigkeitsquelle 9 bereitgestellt. Von der Dialysierflüssigkeitsquelle 9 führt eine Dialysierflüssigkeitszuführleitung 10 zu dem Einlass 4a der Dialysierflüssigkeitskammer 4 des Dialysators 1. Von dem Auslass 4b der Dialysierflüssigkeitskammer 4 führt eine Dialysierflüssigkeitsabführleitung 11 zu einem Abfluss 12. In die Dialysierflüssigkeitszuführleitung 10 ist eine erste Dialysierflüssigkeitspumpe 13 und in die Dialysierflüssigkeitsabführleitung 11 ist eine zweite Dialysierflüssigkeitspumpe 14 geschaltet. Die erste Dialysierflüssigkeitspumpe 13 fördert Dialysierflüssigkeit von der Dialysierflüssigkeitsquelle mit einer bestimmten Dialysierflüssigkeitszuführrate $Q_{di}$ zu dem Einlass 4a der Dialysierflüssigkeitskammer 4, während die zweite Dialysierflüssigkeitspumpe 14 von dem Auslass 4b der Dialysierflüssigkeitskammer 4 Dialysierflüssigkeit mit einer bestimmten Dialysierflüssigkeitsabführrate $Q_{do}$ zu dem Abfluss 12 fördert.

Während der Dialysebehandlung kann Dialysierflüssigkeit aus dem Dialysierflüssigkeitssystem 5B als Substitutionsflüssigkeit über eine Substitutionsflüssigkeitsleitung 15 dem extrakorporalen Blutkreislauf 5A zugeführt werden, die stromauf der ersten Dialysierflüssigkeitspumpe 13 von der Dialysierflüssigkeitszuführleitung 10 abzweigt.

Die Substitutionsflüssigkeitsleitung 15 weist zwei Leitungsabschnitte 15a und 15b auf, von denen der eine Leitungsabschnitt 15a zu der arteriellen Blutleitung 6 und der andere Leitungsabschnitt 15b zu der venösen Blutleitung 7 führt.

Die Substitutionsflüssigkeit wird mittels einer Substitutionspumpe 16, insbesondere Rollenpumpe gefördert, in die die Substitutionsflüssigkeitsleitung 15 eingelegt ist. In die Substitutionsflüssigkeitsleitung 15 ist stromauf der Substitutionspumpe ein in zwei Kammern 17a, 17b unterteiltes Sterilfilter 17 geschaltet. Die Substitutionspumpe zusammen mit den zugehörigen Leitungen und dem Sterilfilter bilden die Substitutionseinrichtung der Dialysevorrichtung. Zum Abklemmen der beiden Leitungsabschnitte 15a, 15b der Substitutionsflüssigkeitsleitung 15 können Absperrorgane, beispielsweise Schlauchklemmen vorgesehen sein, die aber der besseren Übersichtlichkeit halber nicht dargestellt sind.

Die Blutpumpe 8, die erste und zweite Dialysierflüssigkeitspumpe 13 und 14 sowie die Substitutionspumpe 16 sind über Steuerleitungen 8', 13', 14' 16' mit einer zentralen Steuer- und Regeleinheit 18 verbunden, von der die Pumpen unter Berücksichtigung der vorgegebenen Behandlungsparameter angesteuert werden.

Zum Betrieb der Hämo(dia)filtrationsvorrichtung als Hämodialysevorrichtung werden die Blutpumpe 8 sowie die erste und zweite Dialysierflüssigkeitspumpe 13 und 14 betrieben, wobei Dialysierflüssigkeit durch die Dialysierflüssigkeitskammer 4 des Dialysators 1 strömt. Zum Betrieb der Hämo(dia)filtrationsvorrichtung als Hämodiafiltrationsvorrichtung wird die Substitutionspumpe 16 betrieben, so dass über den Sterilfilter 17 sterile Dialysierflüssigkeit als Substitutionsflüssigkeit wahlweise zu der arteriellen Zugabestelle 15A stromab der Blutpumpe 8 und stromauf der Blutkammer 3 (Prädilution) oder zu der venösen Zugabestelle 15B stromab der Blutkammer (Postdilution) strömt. Es ist aber auch ein Betrieb der Hämo(dia)filtrationsvorrichtung nur als Hämofiltrationsvorrichtung möglich, wenn die erste Dialysierflüssigkeitspumpe 13 nicht betrieben wird und somit der Zufluss von Dialysierflüssigkeit in die Dialysierflüssigkeitskammer des Dialysators unterbrochen wird.

Die Vorrichtung zur Bestimmung des Anteils der Fistelrezirkulation und/oder der kardiopulmonalen Rezirkulation an der Rezirkulation ist Bestandteil der extrakorporalen Blutbehandlungsvorrichtung. Die Vorrichtung verfügt über eine Steuereinheit, die Bestandteil der zentralen Steuer- und Recheneinheit 18 der Blutbehandlungsvorrichtung ist.

Weiterhin weist die Vorrichtung eine Einheit 19 zum Bestimmen des relativen Blutvolumens RBV(t) oder einer mit dem Blutvolumen korrelierenden Größe, beispielsweise des Hämatokrits Hct(t) auf. Diese Einheit ist in Fig. 1 nur schematisch dargestellt, da Geräte zum Messen des Blutvolumens oder Hämatokrits dem Fachmann bekannt sind. Es können grundsätzlich alle bekannten Geräte zum Messen dieser Größen eingesetzt werden. Bei dem vorliegenden Ausführungsbeispiel wird das relative Blutvolumen oder der Hämatokrit in dem Blut bestimmt, das durch die arterielle Blutleitung 6 strömt.

Darüber hinaus weist die Vorrichtung eine Einheit 20 zum Bestimmen der Summe von Fistelrezirkulation $R_A$ und kardiopulmonaler Rezirkulation $R_{CP}$ auf. Derartige Geräte sind dem Fachmann ebenfalls bekannt. Beispielsweise kann ein Gerät eingesetzt werden, das die Rezirkulation R nach dem als Thermodilution bekannten Verfahren bestimmt (EDTNA-ERCA Journal 19,6 (1993)). Grundsätzlich können aber auch alle anderen bekannten Geräte zur Bestimmung der Rezirkulation R eingesetzt werden.

Des Weiteren weist die Vorrichtung eine Auswerteinheit 21 auf, die über Datenleitungen 22, 23 mit der Einheit 19 zum Bestimmen des relativen Blutvolumens oder Hämatokrits und mit der Einheit 20 zum Bestimmen der Rezirkulation verbunden ist. Über eine weitere Datenleitung 24 kommuniziert die Auswerteinheit mit der zentralen Steuereinheit 18.

Während der extrakorporalen Blutbehandlung steuert die Steuereinheit 18 die Blutpumpe 8 derart an, dass Blut in die Blutkammer 3 des Dialysators 1 mit der Blutflussrate Qb strömt, und steuert die erste und zweite Dialysierflüssigkeitspumpe 13, 14 derart an, dass Dialysierflüssigkeit mit der Dialysierflüssigkeitsrate Qdi in die Dialysierflüssigkeitskammer 4 strömt und Dialysierflüssigkeit mit der Dialysierflüssigkeitsrate Qdo aus der Dialysierflüssigkeitskammer 4 strömt. Die Steuereinheit 18 steuert die Substitutionspumpe 16 derart an, dass Substitutionsflüssigkeit mit der Substitutionsrate Qs dem Blut wahlweise stromauf (Prädilution) und/oder stromab (Postdilution) der Blutkammer 3 zugeführt wird.

Zunächst wird der Fall der Postdilution beschrieben, bei dem die Substitutionspumpe 16 Substitutionsflüssigkeit mit der Substitutionsrate Qs dem Blut stromab der Blutkammer zuführt. In diesem Fall strömt die Substitutionsflüssigkeit über den Leitungsabschnitt 15b der Substitutionsflüssigkeitsleitung, während der Leitungsabschnitt 15a abgesperrt ist.

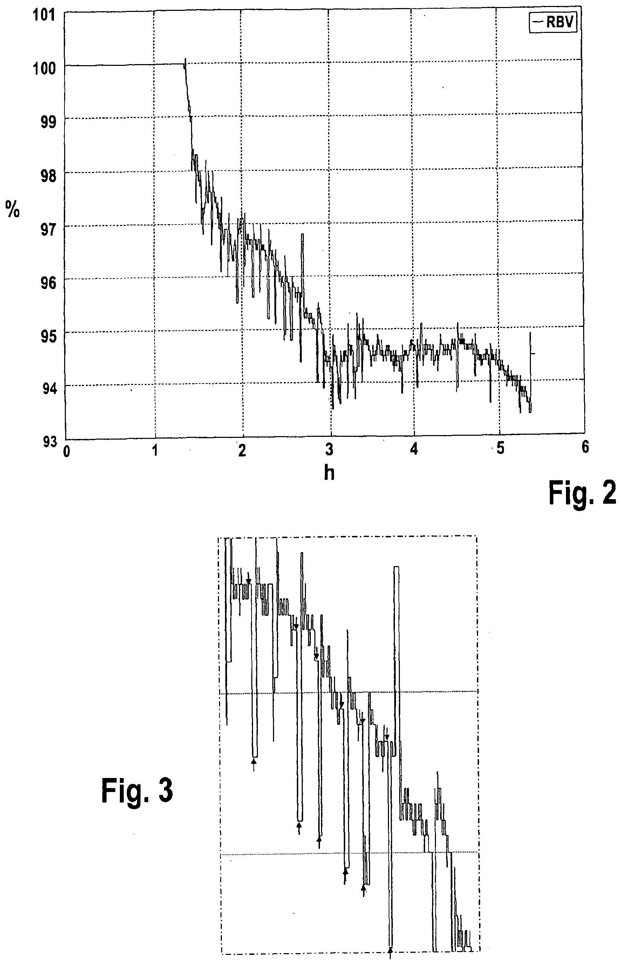

Während der extrakorporalen Blutbehandlung wird mit der Einheit 19 das relative Blutvolumen RBV(t) oder der Hämatokrit Hct(t) fortlaufend überwacht. Fig. 2 zeigt den zeitlichen Verlauf des relativen Blutvolumens während der mehrstündigen Blutbehandlung.

Zur Bestimmung des Anteils der Fistelrezirkulation und/oder der kardiopulmonalen Rezirkulation an der Rezirkulation steuert die Steuereinheit 18 die Substitutionspumpe 16 derart an, dass deren Förderrate vorzugsweise nur für ein vorgegebenes Zeitintervall um einen vorgegebenen Betrag vorzugsweise verringert wird oder die Substitutionspumpe 16 vorzugsweise für ein vorgegebenes Zeitintervall angehalten wird. Gleichzeitig steuert die Steuereinheit 18 die erste und zweite Dialysierflüssigkeitspumpe 13 und 14 derart an, dass die Flussrate $Q_{FM}$, d.h. die Flussrate, mit der Flüssigkeit über die Membran des Dialysators dem extrakorporalen Blutkreislauf entzogen wird, innerhalb des gleichen Zeitintervalls um den gleichen Betrag verringert wird, wie die Substitutionsrate verringert wird, oder der Entzug von Flüssigkeit über die Membran innerhalb des gleichen Zeitintervalls gänzlich unterbrochen wird. Dies ist besonders einfach bei Dialysegeräten zu bewerkstelligen, bei denen die Funktion der beiden Dialysierflüssigkeitspumpen 13 und 14 durch ein 1:1 bilanzierendes System sowie eine getrennte Ultrafiltrationsleitung realisiert werden, wie es beispielsweise bei dem Dialysiergerät der Fall ist, das in der US 4267040 beschrieben ist. In diesem Fall zweigt die Substitutionsflüssigkeitsleitung 15 vorzugsweise stromabwärts des Bilanziersystems von der Dialysierflüssigkeitszuführleitung 10 ab. Wird dann die Substitutionspumpe abgeschaltet, verringert sich auch die Flussrate entsprechend, mit der Flüssigkeit über die Membran des Diaysators dem extrakorporalen Blutkreislauf entzogen wird. Wird dann der Dialysator durch einen nicht gezeigten Bypass zwischen Dialysierzuführ-und -abführleitung abgekoppelt, so kommt es zur Unterbindung jedweder Ultrafiltration im Dialysator.

Fig. 2 zeigt den Fall, dass die Substitutionspumpe 16 für ein vorgegebenes kurzes Zeitintervall abgeschaltet wird und gleichzeitig die erste und zweite Dialysierflüssigkeitspumpe 13, 14 derart angesteuert werden, dass über die Membran des Dialysators Flüssigkeit (Ultrafiltrat) nicht entzogen wird. Das Anhalten der Substituatpumpe bei gleichzeitiger Unterbrechung des Entzugs von Flüssigkeit über die Membran des Dialysators führt zu einer kurzzeitigen Änderung des relativen Blutvolumens RBV(t) oder des Hämatokrits Hct(t). Die Änderung des Blutvolumens RBV(t) ist in Fig. 2 als kurzzeitiger Einbruch (Peak) deutlich zu erkennen. Da die Substitutionspumpe während der Blutbehandlung in vorgegebenen Abständen für ein vorgegebenes Zeitintervall angehalten wird, ergeben sich eine Vielzahl von kurzfristigen Einbrüchen des Blutvolumens.

Fig. 3 zeigt die kurzzeitigen Änderungen des Blutvolumens in Folge des Abschaltens der Substitutionspumpe bei gleichzeitiger Unterbrechung des Entzugs von Ultrafiltrat während eines Abschnitts der Blutbehandlung in vergrößerter Darstellung. Es zeigt sich, dass das relative Blutvolumen RBV(t) immer um den gleichen Betrag abnimmt. Entsprechendes gilt für eine mit dem Blutvolumen korrelierende Größe, beispielsweise den Hämatokrit. Nach dem Abschalten der Substitutionspumpe und der Unterbrechung des Entzugs von Substituat bildet sich im extrakorporalen Kreislauf eine Art verdickte "Blutsäule" aus, die "einen Bolus" darstellt, der zu einer Änderung des Blutvolumens RBV oder des Hämatokrits führt.

Die Abnahme des relativen Blutvolumens bzw. die Zunahme der Dichte des Blutes oder eines Blutinhaltsstoffes ist bei der Postdilution darauf zurückzuführen, dass unmittelbar nach der Verringerung der Substitutionsrate $Q_S$, mit der Substitutionsflüssigkeit dem Blut zugeführt wird, und der gleichzeitigen Verringerung der Flüssigkeitsrate $Q_{FM}$, mit der dem Blut über die Membran des Dialysators oder Filters Flüssigkeit entzogen wird, dem nunmehr aus dem Dialysator strömenden Blut über die Membran noch eine entsprechende Menge an Flüssigkeit entzogen worden ist, ohne dass es zu einer entsprechenden Verdünnung stromab des Dialysators mit Substitutionsflüssigkeit gekommen ist. Dadurch ist das aus dem Dialysator oder Filter fließende Blut unmittelbar nach der Verringerung der Raten $Q_S$ und $Q_{FM}$ eingedickt. Eine Verringerung der Menge der dem Blut nach dem Durchtritt durch den Dialysator zugeführten Substitutionsflüssigkeit (Postdilution) führt daher unmittelbar zu einem Anstieg der Dichte des Bluts oder des Blutinhaltsstoffes im Blutkreislauf stromab des Dialysators. Wenn bei einer Prädilution die Raten $Q_S$ und $Q_{FM}$ hingegen verringert werden, ist das im Dialysator oder Filter befindliche Blut bereits durch den vorherigen Zufluss von Substitutionsflüssigkeit verdünnt worden. Da die dem Substituatfluss entsprechende Filtration im Dialysator verringert wird oder ausbleibt, nimmt die Dichte des zum Patienten zurückzuströmenden Bluts in diesem Fall ab bzw. das relative Blutvolumen nimmt zu.

Die Einheit 19 zum Bestimmen des relativen Blutvolumens bestimmt das relative Blutvolumen ($RBV(t_1)$) vor dem Anhalten und das relative Blutvolumen ($RBV(t_2)$) nach dem Anhalten der Substitutionspumpe und Unterbrechung des Entzugs von Ultrafiltrat. Dabei fällt der Zeitpunkt, zu dem die Zufuhr von Substitutionsflüssigkeit und der Entzug von Ultrafiltrat unterbrochen wird, nicht mit dem Zeitpunkt $t_1$ bzw. $t_2$ zusammen, zu dem das relative Blutvolumen gemessen wird. Die Messung erfolgt vielmehr zu einem Zeitpunkt, der ein bestimmtes Zeitintervall vor bzw. nach der Änderung der obigen Flussraten liegt, da es nach dem Abschalten der Pumpen eine gewisse Zeit dauert, bis die "Blutsäule" am Messpunkt ankommt. Auf der Grundlage des Verhältnisses k von dem Blutvolumen ($RBV(t_2)$)/($RBV(t_1)$) vor und nach dem Anhalten der Substitutionspumpe wird dann die Fistelrezirkulation $R_A$ berechnet, wie nachfolgend im Einzelnen beschrieben wird.

Die einzelnen Größen werden wie folgt bezeichnet:

$R_A$ :     Rezirkulation in Shunt/Fistel
$Hct_P$ :     Hämatokrit des Dialysepatienten
$Hct_A$ :     Hämatokrit in der arteriellen Blutleitung zwischen arterieller Nadel und Blutpumpe
$Hct_V$ :     Hämatokrit in der venösen Blutleitung zwischen venöser Tropfkammer und venöser Nadel
BPR :     Förderrate der Blutpumpe
UFR :     Netto-Ultrafiltrationsrate
$Q_S$ :     Förderrate der Substitutionspumpe
$\alpha$ :     Verhältnis der UFR zur Förderrate der Blutpumpe
$\beta_1$ :     Berechnungsfaktor bei Post-Dilution
$\beta_2$ :     Berechnungsfaktor bei Pre-Dilution

RBV : relatives Blutvolumen

$Q_{FM}$ Flussrate, mit der Flüssigkeit über die Membran des Dialysators dem Blut entzogen wird

Es gilt: $Q_{FM} = Q_S + UFR$

Zunächst wird der Fall der Postdilution betrachtet. Es sei angenommen, dass zum Zeitpunkt $t_1$ die Blutpumpe 8 mit der Förderrate BPR Blut durch den extrakorporalen Kreislauf 5A pumpt, während die Substitutionspumpe 16 dem Blut im extrakorporalen Kreislauf mit der Förderrate $Q_S$ Substitutionsflüssigkeit stromab der Blutkammer 3 des Dialysators 1 oder Filters zuführt und über die Membran 2 des Dialysators Flüssigkeit mit der Flussrate $Q_S$ entzogen wird.

$$Hct_A(t_1) = Hct_P(t_1) \cdot (1 - R_A(t_1)) + Hct_V(t_1) \cdot R_A(t_1) \tag{1}$$

$$Hct_V(t_1) = \frac{Hct_A(t_1)}{1 - \alpha} \tag{2}$$

$$\alpha = \frac{UFR(t_1)}{BPR(t_1)} \tag{3}$$

$$Hct_A(t_1) = Hct_P(t_1) \cdot (1 - R_A(t_1)) \cdot \frac{1 - \alpha}{1 - \alpha - R_A(t_1)} \tag{4}$$

Nunmehr wird angenommen, dass die Substitutionspumpe zum Zeitpunkt $t_2$ angehalten und gleichzeitig der Entzug von Flüssigkeit über die Membran des Dialysators unterbrochen wird. Die Förderrate BPR der Blutpumpe bleibt hingegen unverändert ($RBV(t_1) = RBV(t_2)$).

$$Hct_A(t_2) = Hct_P(t_2) \cdot (1 - R_A(t_2)) \cdot \frac{1 - \beta}{1 - \beta - R_A(t_2)} \tag{5}$$

$$\beta = \frac{UFR(t) + Q_S(t)}{BPR(t)} \tag{6}$$

Aus Gleichung (4) und (5) ergibt sich:

$$\frac{Hct_A(t_1)}{Hct_A(t_2)} = \frac{(1 - \alpha) \cdot (1 - \beta - R_A(t_2))}{(1 - \beta) \cdot (1 - \alpha - R_A(t_1))} \cdot \frac{Hct_P(t_1) \cdot (1 - R_A(t_1))}{Hct_P(t_2) \cdot (1 - R_A(t_2))} \tag{7}$$

Unter der Annahme, dass sich der Hämatokrit des Patienten und die Rezirkulation nicht ändern, d.h. $Hct_p(t_1) = Hct_p(t_2) = R(t_1) = R(t_2) = R$ ergibt sich mit

$$k = Hct_A(t_1) / Hct_A(t_2) = RBV(t_2)/RBV(t_1) < 1$$

$$k = \frac{(1 - \alpha) \cdot (1 - \beta - R_A)}{(1 - \beta) \cdot (1 - \alpha - R_A)} \tag{8}$$

Nach Umformung der Gleichung (8) ergibt sich:

$$R_A = \frac{(1-\alpha)\cdot(1-\beta)\cdot(1-k)}{(1-\alpha)-k\cdot(1-\beta)} \text{ mit} \tag{9}$$

$$\alpha = \frac{UFR(t)}{BPR(t)}$$

und

$$\beta = \frac{UFR(t)+Q_S(t)}{BPR(t)}$$

Für den Fall der Prädilution ergibt sich:

$$R_A = \frac{(1-\alpha)\cdot(1-\beta)\cdot(1-k)}{(1-\alpha)-k\cdot(1-\beta)} \text{ mit} \tag{10}$$

$$\alpha = \frac{UFR(t)}{BPR(t)} \text{ und} \tag{3}$$

$$\beta = \frac{UFR(t)-Q_S(t)}{BPR(t)} \tag{11}$$

$$k = Hct_A(t_1)/\ Hct_A(t_2) = RBV(t_2)/RBV(t_1) > 1 \tag{12}$$

Die Auswerteinheit 21 berechnet die Rezirkulation also für den Fall der Postdilution nach Gleichung (9) und für den Fall der Prädilution nach Gleichung (10) aus der Ultrafiltrationsrate UFR und der Förderrate der Blutpumpe BPR und der Förderrate $Q_S$ der Substitutionspumpe sowie dem Verhältnis k des mit der Einheit 19 zum Zeitpunkt $t_1$ vor der Unterbrechung der Substitution und Ultrafiltration und zum Zeitpunkt $t_2$ nach der Unterbrechung der Substitution und Ultrafiltration gemessenen Hämatokrits $Hct_A$ ($t_1$) und $Hct_A$ ($t_2$) des Bluts in der arteriellen Blutleitung.

Anstelle des Hämatokrits kann der Koeffizient k auch aus dem Quotienten des relativen Blutvolumens $RBV(t_2)$ zum Zeitpunkt $t_2$ nach der Unterbrechung der Substitution und Ultrafiltration und dem relativen Blutvolumen $RBV(t_1)$ zum Zeitpunkt $t_1$ vor der Unterbrechung der Substitution und Ultrafiltration berechnet werden. Folglich kommt es nur auf das Verhältnis des relativen Blutvolumens vor und nach der Unterbrechung oder des Verhältnisses einer mit dem Blutvolumen korrelierenden Größe, beispielsweise des Hämatokrits an.

Zur Bestimmung des Anteils der Fistelrezirkulation $R_A$ an der gesamten Rezirkulation R hält die Steuereinheit 18 die Substitutionspumpe 16 für ein vorgegebenes Zeitintervall an ($Q_S$ = 0) und stellt die Förderraten der Dialysierflüssigkeitspumpen 13 und 14 derart ein, dass über die Membran des Dialysators dem Blut keine Flüssigkeit innerhalb des vorgegebenen Zeitintervalls entzogen wird ($Q_{FM}$ = 0). Daraus folgt, dass die Ultrafiltration unterbrochen ist (UFR = 0). Dabei können auch die Förderraten der Dialysierflüssigkeitspumpen 13 und 14 gleich null eingestellt werden ($Q_{di}$ = $Q_{do}$=0). Ein solcher Zustand stellt sich beispielsweise ein, wenn der Dialysator im Rahmen eines Druckhaltetest durch einen Bypass überbrückt wird. Nach Ablauf des Zeitintervalls stellt die Steuereinheit 18 wieder die ursprünglichen Förderraten für die Pumpen ein.

Vor und nach der Unterbrechung der Substitution und der Ultrafiltration bestimmt die Einheit 19 das relative Blutvolumen $RBV(t_1)$ und $RBV(t_2)$ oder den Hämatokrit $Hct_A(t_1)$ und $Hct_A$ ($t_2$). Die Auswerteinheit 21 berechnet aus den Werten des relativen Blutvolumens oder des Hämatokrits den Koeffizienten k. Darüber hinaus berechnet die Auswerteinheit aus der Netto-Ultrafiltrationsrate UFR vor der Unterbrechung der Substitution und Ultrafiltration und der vor und nach der Unterbrechung der Substitution und Ultrafiltration gleich bleibenden Förderrate der Blutpumpe BPR den Ko-

effizienten $\alpha$ nach Gleichung (3). Aus der Netto-Ultrafiltrationsrate UFR und der Substitutionsrate $Q_S$ und der Förderrate BPR der Blutpumpe berechnet die Recheneinheit nach Gleichung (6) den Koeffizienten $\beta$. Die Fistelrezirkulation $R_A$ berechnet die Recheneinheit dann nach Gleichung (9) für den Fall der Postdilution.

Für den Fall der Prädilution berechnet die Recheneinheit den Koeffizienten $\alpha$ nach Gleichung (3) und den Koeffizienten $\beta$ nach Gleichung (11). Die Fistelrezirkulation $R_A$ für den Fall der Prädilution berechnet die Recheneinheit dann nach Gleichung (10).

Da die Messung der Rezirkulation sehr schnell erfolgt, wird nur die Fistelrezirkulation $R_A$, nicht aber die Summe R von Fistel- und kardiopulmonaler Rezirkulation erfasst. Die Summe R von Fistelrezirkulation $R_A$ und kardiopulmonaler Rezirkulation $R_{CP}$ bestimmt die Einheit 20 nach den bekannten Verfahren, beispielsweise nach dem als Thermodilution bekannten Verfahren (EDTNA-ERCA Journal 19,6 (1993).

Daraufhin berechnet die Auswerteinheit 21 den Anteil der Fistelrezirkulation $R_A$ an der Summe R von Fistel- und kardiopulmonaler Rezirkulation. Die kardiopulmonale Rezirkulation $R_{CP}$ wird in der Auswerteinheit dadurch berechnet, dass die Differenz zwischen der gemessenen Summe R von Fistel- und kardiopulmonaler Rezirkulation und der berechneten Fistelrezirkulation $R_A$ bestimmt wird.

Die Rezirkulationswerte können auf einer nicht dargestellten Anzeigeeinheit angezeigt werden und/oder zur Berechnung weiterer für die extrakorporale Blutbehandlung charakteristischer Größen herangezogen werden.

**Patentansprüche**

1. Vorrichtung zur Bestimmung des Anteils der Rezirkulation in einer Fistel ($R_A$) und / oder der kardiopulmonalen Rezirkulation ($R_{CP}$) an der Summe von Fistelrezirkulation ($R_A$) und kardiopulmonaler Rezirkulation ($R_{CP}$) für eine Vorrichtung zur extrakorporalen Blutbehandlung, wobei die extrakorporale Blutbehandlungsvorrichtung aufweist:

   einen extrakorporalen Blutkreislauf (5A), der eine erste Kammer (3) eines durch eine Membran (2) in die erste Kammer und eine zweite Kammer (4) unterteilten Dialysators (1) oder Filters einschließt, und ein Flüssigkeitssystem (5B), das die zweite Kammer des Dialysators oder Filters einschließt,
   eine Einrichtung (8) zum Zuführen von Blut in die erste Kammer des Dialysators oder Filters mit einer bestimmten Blutflussrate,
   eine Substitutionseinrichtung (16) zum Zuführen von Substitutionsflüssigkeit dem Blut stromauf oder stromab des Dialysators oder Filters mit einer bestimmten Substitutionsrate und
   eine Ultrafiltrationseinrichtung (13, 14) zum Entziehen, von Flüssigkeit dem Blut über die Membran des Dialysators oder Filters mit einer bestimmten Flussrate,
   **dadurch gekennzeichnet, dass**
   die Vorrichtung zur Bestimmung des Anteils der Rezirkulation in einer Fistel ($R_A$) und / oder der kardiopulmonalen Rezirkulation ($R_{CP}$) an der Summe von Fistelrezirkulation ($R_A$) und/oder kardiopulmonaler Rezirkulation ($R_{CP}$) aufweist:

      eine Steuereinheit (18) zum Steuern der Substitutionseinrichtung (16) und der Ultrafiltrationseinrichtung (13, 14), wobei die Steuereinheit (18) derart ausgebildet ist, dass stromauf oder stromab des Dialysators oder Filters die Substitutionsrate $Q_S$ um einen vorgegebenen Betrag verändert wird, während die Flussrate $Q_{FM}$ der durch die Membran des Dialysators entzogenen Flüssigkeit verändert wird,
      eine Einheit (19) zum Bestimmen des Blutvolumens ($RBV(t_1)$); ($RBV(t_2)$) oder einer mit dem Blutvolumen korrelierenden Größe vor und nach der Veränderung der Substitutionsrate und der Flussrate der durch die Membran des Dialysators entzogenen Flüssigkeit;
      eine Einheit (20) zum Bestimmen der Summe von Fistelrezirkulation ($R_A$) und kardiopulmonaler Rezirkulation ($R_{CP}$); und
      eine Auswerteinheit (21), die derart ausgebildet ist, dass

   aus dem ermittelten Blutvolumen oder der mit dem Blutvolumen korrelierenden Größe vor und nach der Veränderung der Substitutionsrate $Q_S$ und der Flussrate $Q_{FM}$ der durch die Membran des Dialysators oder Filters entzogenen Flüssigkeit die Fistelrezirkulation ($R_A$) bestimmt wird, und
   aus der ermittelten Fistelrezirkulation ($R_A$) und der ermittelten Summe von Fistelrezirkulation ($R_A$) und kardiopulmonaler Rezirkulation ($R_{CP}$) der Anteil der Fistelrezirkulation ($R_A$) und/oder der kardiopulmonalen Rezirkulation ($R_{CP}$) an der Summe von Fistelrezirkulation ($R_A$) und/oder kardiopulmonaler Rezirkulation ($R_{CP}$) bestimmt wird.

2. Vorrichtung nach Anspruch 1, **dadurch gekennzeichnet, dass** die Steuereinheit (18) derart ausgebildet ist, dass die Substitutionsrate $Q_S$ und die Flussrate $Q_{FM}$ der durch die Membran des Dialysators oder Filters entzogenen Flüssigkeit um den gleichen Betrag verringert wird.

3. Vorrichtung nach Anspruch 1 oder 2, **dadurch gekennzeichnet, dass** die Steuereinheit (18) derart ausgebildet ist, dass die Zufuhr von Substitutionsflüssigkeit und der Entzug von Flüssigkeit über die Membran des Dialysators oder Filters unterbrochen wird.

4. Vorrichtung nach Anspruch 3, **dadurch gekennzeichnet, dass** die Steuereinheit (18) derart ausgebildet ist, dass die Zufuhr von Substitutionsflüssigkeit und der Entzug von Flüssigkeit über die Membran des Dialysators oder Filters für ein vorgegebenes Zeitintervall unterbrochen wird.

5. Vorrichtung nach einem der Ansprüche 1 bis 4 für eine extrakorporale Blutbehandlungsvorrichtung, bei der die Substitutionseinrichtung derart ausgebildet ist, dass dem Blut stromab der ersten Kammer des Dialysators oder Filters Substitutionsflüssigkeit mit einer bestimmten Substitutionsrate Qs zugeführt und dem Blut mit einer bestimmten Flussrate $Q_{FM}$ über die Membran des Dialysators oder Filters Flüssigkeit entzogen wird, so dass dem Patienten Flüssigkeit mit einer bestimmten Ultrafiltrationsrate UFR Flüssigkeit entzogen wird, **dadurch gekennzeichnet, dass** die Auswerteinheit (21) derart ausgebildet ist, dass der Quotient aus der Flussrate $Q_S$ und der Blutflussrate BPR als ein erster Koeffizient $\alpha$ berechnet wird, und als ein zweiter Koeffizient $\beta$ der Quotient aus der Summe von der Ultrafiltrationsrate UFR und der Substitutionsrate $Q_S$ und der Blutflussrate BPR berechnet wird, wobei auf der Grundlage des Verhältnisses k von dem Blutvolumen RBV oder der mit dem Blutvolumen korrelierenden Größe vor und nach der Veränderung der Substitutionsrate $Q_S$ und der Flussrate $Q_{FM}$ der durch die Membran des Dialysators oder Filters entzogenen Flüssigkeit und auf der Grundlage des ersten und zweiten Koeffizienten $\alpha$, $\beta$ die Fistelrezirkulation $R_A$ bestimmt wird.

6. Vorrichtung nach Anspruch 5, **dadurch gekennzeichnet, dass** die Auswerteinheit (21) derart ausgebildet ist, dass die Fistelrezirkulation $R_A$ aus dem Verhältnis k von dem Blutvolumen RBV oder der mit dem Blutvolumen korrelierenden Größe vor und nach der Veränderung der Substitutionsrate $Q_S$ und der Flussrate $Q_{FM}$ der durch die Membran des Dialysators oder Filters entzogenen Flüssigkeit und aus dem ersten und zweiten Koeffizienten $\alpha$, $\beta$ nach der folgenden Gleichung berechnet wird:

$$R_A = \frac{(1-\alpha)\cdot(1-\beta)\cdot(1-k)}{(1-\alpha)-k\cdot(1-\beta)} \ .$$

7. Vorrichtung einem der Ansprüche 1 bis 4 für eine extrakorporale Blutbehandlungsvorrichtung, bei der die Substitutionseinrichtung derart ausgebildet ist, dass dem Blut stromauf der ersten Kammer des Dialysators oder Filters Substitutionsflüssigkeit mit einer bestimmten Substitutionsrate $Q_S$ zugeführt und dem Blut mit einer bestimmten Flussrate $Q_{FM}$ über die Membran des Dialysators oder Filters Flüssigkeit entzogen wird, so dass dem Patienten Flüssigkeit mit einer bestimmten Ultrafiltrationsrate UFR Flüssigkeit entzogen wird, **dadurch gekennzeichnet**, die Auswerteinheit (21) derart ausgebildet ist, dass der Quotient aus der Flussrate Qs und der Blutflussrate BPR als ein erster Koeffizient $\alpha$ berechnet wird, und als ein zweiter Koeffizient $\beta$ der Quotient aus der Differenz von der Ultrafiltrationsrate UFR und der Substitutionsrate $Q_S$ und der Blutflussrate BPR berechnet wird, wobei auf der Grundlage des Verhältnisses k von dem Blutvolumen RBV oder der mit dem Blutvolumen korrelierenden Größe vor und nach der Veränderung der Substitutionsrate $Q_S$ und der Flussrate $Q_{FM}$ der durch die Membran des Dialysators oder Filters entzogenen Flüssigkeit und auf der Grundlage des ersten und zweiten Koeffizienten $\alpha$, $\beta$ die Fistelrezirkulation $R_A$ bestimmt wird.

8. Vorrichtung nach Anspruch 7, **dadurch gekennzeichnet, dass** die Auswerteinheit (21) derart ausgebildet ist, dass die Fistelrezirkulation $R_A$ aus dem Verhältnis k von dem Blutvolumen RBV oder der mit dem Blutvolumen korrelierenden Größe vor und nach der Veränderung der Substitutionsrate $Q_S$ und der Flussrate $Q_{FM}$ der durch die Membran des Dialysators oder Filters entzogenen Flüssigkeit und aus dem ersten und zweiten Koeffizienten $\alpha$, $\beta$ nach der folgenden Gleichung berechnet wird:

$$R_A = \frac{(1-\alpha)\cdot(1-\beta)\cdot(1-k)}{(1-\alpha)-k\cdot(1-\beta)}\ .$$

**9.** Vorrichtung nach einem der Ansprüche 1 bis 8, **dadurch gekennzeichnet, dass** die Auswerteinheit (21) derart ausgebildet ist, dass die kardiopulmonare Rezirkulation $R_{CP}$ aus der Differenz der ermittelten Summe R von Fistelrezirkulation $R_A$ und kardiopulmonaler Rezirkulation Rcp und der ermittelten Fistelrezirkulation $R_A$ berechnet wird.

**10.** Blutbehandlungsvorrichtung mit einer Vorrichtung nach einem der Ansprüche 1 bis 9 zur Bestimmung des Anteils der Rezirkulation in einer Fistel ($R_A$) und / oder der kardiopulmonalen Rezirkulation ($R_{CP}$) an der Summe von Fistelrezirkulation ($R_A$) und kardiopulmonaler Rezirkulation ($R_{CP}$)+

**11.** Blutbehandlungsvorrichtung nach Anspruch 10, **dadurch gekennzeichnet, dass** die Blutbehandlungsvorrichtung aufweist:

einen Dialysator (1) oder Filter, der durch eine Membran (2) in eine erste Kammer (3) und eine zweite Kammer (4) unterteilt ist,
einen extrakorporalen Blutkreislauf (5A), der eine zu der ersten Kammer (3) des Dialysators (1) oder Filters führende Blutzuführleitung (6) und eine von der ersten Kammer des Dialysators oder Filters abgehende Blutabführleitung (7) aufweist,
ein Flüssigkeitssystem (SB), das die zweite Kammer (4) des Dialysators (1) oder Filters einschließt,
eine Substitutionseinrichtung (16) mit einer zu einer ersten Zugabestelle (15A) an der Blutzuführleitung (6) führenden ersten Substituatleitung (15, 15a) zum Zuführen von Substitutionsflüssigkeit stromauf des Dialysators (1) oder Filters und/oder einer zu einer zweiten Zugabestelle (15B) an der Blutabführleitung (7) führenden zweiten Substituatleitung (15, 15b) zum Zuführen von Substitutionsflüssigkeit stromab des Dialysators oder Filters,
eine Ultrafiltrationseinrichtung (13, 14), mit der dem Blut Flüssigkeit über die Membran (2) des Dialysators (1) entzogen werden kann.

## Claims

**1.** A device for determining the share of the recirculation in a fistula ($R_A$) and/or the cardiopulmonary recirculation (($R_{CP}$) in the sum of fistula recirculation ($R_A$) and cardiopulmonary recirculation (($R_{CP}$) for an apparatus for extracorporeal blood treatment, the extracorporeal blood treatment apparatus comprising:

an extracorporeal blood circuit (5A), which includes a first chamber (3) of a dialyser (1) or filter divided by a membrane (2) into the first chamber and a second chamber (4), and a fluid system (5B) which includes the second chamber of the dialyser or filter,
a device (8) for supplying blood at a specific blood flow rate into the first chamber of the dialyser or filter,
a substitution device (16) for supplying substitution fluid at a specific substitution rate to the blood upstream or downstream of the dialyser or filter and
an ultrafiltration device (13, 14) for withdrawing fluid at a specific flow rate from the blood via the membrane of the dialyser or filter,
**characterised in that**
the device for determining the share of the recirculation in a fistula ($R_A$) and/or the cardiopulmonary recirculation (($R_{CP}$) in the sum of fistula recirculation ($R_A$) and/or cardiopulmonary recirculation (($R_{CP}$) comprises:

a control unit (18) for controlling the substitution device (16) and the ultrafiltration device (13, 14), the control unit (18) being designed in such a way that substitution rate Qs is changed by a predetermined amount upstream or downstream of the dialyser or filter, while flow rate $Q_{FM}$ of the fluid withdrawn through the membrane of the dialyser is changed,
a unit (19) for determining the blood volume ($RBV(t_1)$); ($RBV(t_2)$) or a quantity correlating with the blood volume before and after the change in the substitution rate and the flow rate of the fluid withdrawn through the membrane of the dialyser;
a unit (20) for determining the sum of fistula recirculation ($R_A$) and cardiopulmonary recirculation (($R_{CP}$); and

an evaluation unit (21), which is designed in such a way that

fistula recirculation ($R_A$) is determined from the ascertained blood volume or the quantity correlating with the blood volume before and after the change in substitution rate $Q_S$ and flow rate $Q_{FM}$ of the fluid withdrawn through the membrane of the dialyser or filter, and

the share of the fistula recirculation ($R_A$) and/or the cardiopulmonary recirculation (($R_{CP}$) in the sum of fistula recirculation ($R_A$) and cardiopulmonary recirculation (($R_{CP}$) is determined from the ascertained fistula recirculation ($R_A$) and the ascertained sum of fistula recirculation ($R_A$) and cardiopulmonary recirculation (($R_{CP}$).

2. The device according to claim 1, **characterised in that** the control unit (18) is designed in such a way that substitution rate Qs and flow rate $Q_{FM}$ of the fluid withdrawn through the membrane of the dialyser or filter are reduced by the same amount.

3. The device according to claim 1 or 2, **characterised in that** the control unit (18) is designed in such a way that the supply of substitution fluid and the withdrawal of fluid via the membrane of the dialyser or filter are interrupted.

4. The device according to claim 3, **characterised in that** the control unit (18) is designed in such a way that the supply of substitution fluid and the withdrawal of fluid via the membrane of the dialyser or filter are interrupted for a predetermined time interval.

5. The device according to any one of claims 1 to 4 for an extracorporeal blood treatment apparatus in which the substitution device is designed in such a way that substitution fluid is fed at a specific substitution rate Qs to the blood *downstream* of the first chamber of the dialyser or filter and fluid is withdrawn at a specific flow rate $Q_{FM}$ from the blood via the membrane of the dialyser or filter, so that fluid is withdrawn from the patient at a specific ultrafiltration rate UFR, **characterised in that** the evaluation unit (21) is designed in such a way that the quotient of flow rate $Q_S$ and blood flow rate BPR is calculated as a first coefficient $\alpha$, and the quotient of the sum of ultrafiltration rate UFR and substitution rate $Q_S$ and blood flow rate BPR is calculated as a second coefficient $\beta$, fistula recirculation $R_A$ being determined on the basis of ratio k of blood volume RBV or the quantity correlating with the blood volume before and after the change in substitution rate Qs and flow rate $Q_{FM}$ of the fluid withdrawn through the membrane of the dialyser or filter and on the basis of first and second coefficients $\alpha$, $\beta$.

6. The device according to claim 5, **characterised in that** the evaluation unit (21) is designed in such a way that fistula recirculation $R_A$ is calculated from ratio k of blood volume RBV or the quantity correlating with the blood volume before and after the change in substitution rate $Q_S$ and flow rate $Q_{FM}$ of the fluid withdrawn through the membrane of the dialyser or filter and from first and second coefficients $\alpha$, $\beta$ according to the following equation:

$$R_A = \frac{(1-\alpha)\cdot(1-\beta)\cdot(1-k)}{(1-\alpha)-k\cdot(1-\beta)}$$

7. The device according to any one of claims 1 to 4 for an extracorporeal blood treatment apparatus in which the substitution device is designed in such a way that substitution fluid is fed at a specific substitution rate Qs to the blood *upstream* of the first chamber of the dialyser or filter and fluid is withdrawn at a specific flow rate $Q_{FM}$ from the blood via the membrane of the dialyser or filter, so that fluid is withdrawn from the patient at a specific ultrafiltration rate UFR, **characterised in that** the evaluation unit (21) is designed in such a way that the quotient of flow rate Qs and blood flow rate BPR is calculated as a first coefficient $\alpha$, and the quotient from the difference in ultrafiltration rate UFR and substitution rate Qs and blood flow rate BPR is calculated as a second coefficient $\beta$, fistula recirculation $R_A$ being determined on the basis of ratio k of blood volume RBV or the quantity correlating with the blood volume before and after the change in substitution rate $Q_S$ and flow rate $Q_{FM}$ of the fluid withdrawn through the membrane of the dialyser or filter and on the basis of first and second coefficients $\alpha$, $\beta$.

8. The device according to claim 7, **characterised in that** the evaluation unit (21) is designed in such a way that fistula recirculation $R_A$ is calculated from ratio k of blood volume RBV or the quantity correlating with the blood volume before and after the change in substitution rate $Q_S$ and flow rate $Q_{FM}$ of the fluid withdrawn through the membrane of the dialyser or filter and from first and second coefficients $\alpha$, $\beta$ according to the following equation:

$$R_A = \frac{(1-\alpha)\cdot(1-\beta)\cdot(1-k)}{(1-\alpha)-k\cdot(1-\beta)}$$

9. The device according to any one of claims 1 to 8, **characterised in that** the evaluation unit (21) is designed in such a way that cardiopulmonary recirculation $R_{CP}$ is calculated from the difference in the ascertained sum R of fistula recirculation $R_A$ and cardiopulmonary recirculation $R_{CP}$ and ascertained fistula recirculation $R_A$.

10. A blood treatment apparatus with a device according to any one of claims 1 to 9 for determining the share of the recirculation in a fistula ($R_A$) and/or the cardiopulmonary recirculation (($R_{CP}$) in the sum of fistula recirculation ($R_A$) and cardiopulmonary recirculation (($R_{CP}$).

11. The blood treatment apparatus according to claim 10, **characterised in that** the blood treatment apparatus comprises:

a dialyser (1) or filter, which is divided by a membrane (2) into a first chamber (3) and a second chamber (4), an extracorporeal blood circuit (5A), which comprises a blood supply line (6) leading to the first chamber (3) of the dialyser (1) or filter and a blood discharge line (7) leading away from the first chamber of the dialyser or filter, a fluid system (5B) which includes the second chamber (4) of the dialyser (1) or filter, a substitution device (16) with a first substituate line (15, 15a) for supplying substitution fluid upstream of the dialyser (1) or filter, said first substituate line leading to a first supply point (15A) on the blood supply line (6), and/or a second substituate line (15, 15b) for supplying substitution fluid downstream of the dialyser or filter, said second substituate line leading to a second supply point (15B) on the blood discharge line (7), an ultrafiltration device (13, 14), with which fluid can be withdrawn from the blood via the membrane (2) of the dialyser (1).

**Revendications**

1. Dispositif de détermination de la fraction de recirculation dans une fistule ($R_A$) et/ou de recirculation cardiopulmonaire ($R_{CP}$) par rapport à la somme de recirculation dans la fistule ($R_A$) et de recirculation cardiopulmonaire ($R_{CP}$) pour un dispositif de traitement extracorporel du sang, dans lequel le dispositif de traitement extracorporel du sang présente :

une circulation sanguine extracorporelle (5A) qui inclut une première chambre (3) d'un dialyseur (1) ou filtre subdivisé par une membrane (2) en la première chambre et une seconde chambre (4), et un système de liquide (5B) qui inclut la seconde chambre du dialyseur ou filtre, un ensemble (8) d'acheminement de sang dans la première chambre du dialyseur ou filtre avec un débit sanguin déterminé, un ensemble de substitution (16) pour l'acheminement de liquide de substitution au sang en amont ou en aval du dialyseur ou filtre avec un taux de substitution déterminé et un ensemble d'ultrafiltration (13, 14) pour le retrait de liquide du sang par l'intermédiaire de la membrane du dialyseur ou filtre avec un débit déterminé, **caractérisé en ce que** le dispositif de détermination de la fraction de recirculation dans une fistule ($R_A$) et/ou de recirculation cardiopulmonaire ($R_{CP}$) par rapport à la somme de recirculation dans la fistule ($R_A$) et/ou de recirculation cardiopulmonaire ($R_{CP}$) présente :

une unité de commande (18) pour la commande de l'ensemble de substitution (16) et de l'ensemble d'ultrafiltration (13, 14), dans lequel l'unité de commande (18) est conçue de telle sorte que, en amont ou en aval du dialyseur ou filtre, le taux de substitution Qs soit modifié d'une quantité prédéterminée, tandis que le débit $Q_{FM}$ du liquide retiré par la membrane du dialyseur est modifié, une unité (19) de détermination du volume sanguin ($RBV(t_1)$ ; $RBV(t_2)$) ou d'une grandeur corrélée au volume sanguin avant et après la modification du taux de substitution et du débit du liquide retiré par la membrane du dialyseur ; une unité (20) de détermination de la somme de recirculation dans la fistule ($R_A$) et de recirculation cardiopulmonaire ($R_{CP}$) ; et une unité d'analyse (21) qui est conçue de telle sorte que

à partir du volume sanguin défini ou de la grandeur corrélée au volume sanguin avant et après la modification du taux de substitution $Q_S$ et du débit $Q_{FM}$ du liquide retiré par la membrane du dialyseur ou filtre, la recirculation dans la fistule ($R_A$) soit déterminée, et

à partir de la recirculation dans la fistule ($R_A$) définie et de la somme définie de la recirculation dans la fistule ($R_A$) et recirculation cardiopulmonaire ($R_{CP}$), la fraction de recirculation dans la fistule ($R_A$) et/ou de recirculation cardiopulmonaire ($R_{CP}$) par rapport à la somme de recirculation dans la fistule ($R_A$) et/ou de recirculation cardiopulmonaire ($R_{CP}$) soit déterminée.

**2.** Dispositif selon la revendication 1, **caractérisé en ce que** l'unité de commande (18) est conçue de telle sorte que le taux de substitution $Q_S$ et le débit $Q_{FM}$ du liquide retiré par la membrane du dialyseur ou filtre soient réduits de la même quantité.

**3.** Dispositif selon la revendication 1 ou 2, **caractérisé en ce que** l'unité de commande (18) est conçue de telle sorte que l'acheminement de liquide de substitution et le retrait de liquide par l'intermédiaire de la membrane du dialyseur ou filtre soit interrompu.

**4.** Dispositif selon la revendication 3, **caractérisé en ce que** l'unité de commande (18) est conçue de telle sorte que l'acheminement de liquide de substitution et le retrait de liquide par l'intermédiaire de la membrane du dialyseur ou filtre soit interrompu pendant un intervalle temporel prédéterminé.

**5.** Dispositif selon l'une quelconque des revendications 1 à 4 pour un dispositif de traitement extracorporel du sang, dans lequel l'ensemble de substitution est conçu de telle sorte que du liquide de substitution soit acheminé au sang en aval de la première chambre du dialyseur ou filtre avec un taux de substitution $Q_S$ déterminé et du liquide soit retiré du sang avec un débit déterminé $Q_{FM}$ par l'intermédiaire de la membrane du dialyseur ou filtre, de sorte que du liquide soit retiré du patient avec un taux d'ultrafiltration UFR déterminé, **caractérisé en ce que** l'unité d'analyse (21) est conçue de telle sorte que le quotient soit calculé à partir du débit $Q_S$ et du débit sanguin BPR comme premier coefficient $\alpha$, et que comme second coefficient $\beta$, le quotient soit calculé à partir de la somme du taux d'ultrafiltration UFR et du taux de substitution $Q_S$ et du débit sanguin BPR, dans lequel, sur la base du rapport k du volume sanguin RBV ou de la grandeur corrélée avec le volume sanguin avant et après la modification du taux de substitution $Q_S$ et du débit $Q_{FM}$ du liquide retiré par la membrane du dialyseur ou filtre et sur la base des premier et second coefficients $\alpha$, $\beta$, la recirculation dans la fistule $R_A$ est déterminée.

**6.** Dispositif selon la revendication 5, **caractérisé en ce que** l'unité d'analyse (21) est conçue de telle sorte que la recirculation dans la fistule $R_A$ soit calculée à partir du rapport k du volume sanguin RBV ou de la grandeur corrélée au volume sanguin avant et après la modification du taux de substitution $Q_S$ et du débit $Q_{FM}$ du liquide retiré par la membrane du dialyseur ou filtre et à partir des premier et second coefficients $\alpha$, $\beta$, selon l'équation suivante :

$$R_A = \frac{(1-\alpha)\cdot(1-\beta)\cdot(1-k)}{(1-\alpha)-k\cdot(1-\beta)} \; .$$

**7.** Dispositif selon l'une quelconque des revendications 1 à 4 pour un dispositif de traitement extracorporel du sang, dans lequel l'ensemble de substitution est conçu de telle sorte que du liquide de substitution soit acheminé au sang en amont de la première chambre du dialyseur ou filtre avec un taux de substitution $Q_S$ déterminé et du liquide soit retiré du sang avec un débit $Q_{FM}$ déterminé par l'intermédiaire de la membrane du dialyseur ou filtre, de sorte que du fluide soit retiré du patient avec un taux d'ultrafiltration UFR déterminé, **caractérisé en ce que** l'unité d'analyse (21) est conçue de telle sorte que le quotient soit calculé à partir du débit $Q_S$ et du débit sanguin BPR comme premier coefficient $\alpha$, et que comme second coefficient $\beta$, le quotient soit calculé à partir de la différence du taux d'ultrafiltration UFR et du taux de substitution Qs et du débit sanguin BPR, dans lequel, sur la base du rapport k du volume sanguin RBV ou de la grandeur corrélée avec le volume sanguin avant et après la modification du taux de substitution $Q_S$ et du débit $Q_{FM}$ du liquide retiré par la membrane du dialyseur ou filtre et sur la base des premier et second coefficients $\alpha$, $\beta$, la recirculation dans la fistule $R_A$ est déterminée.

**8.** Dispositif selon la revendication 7, **caractérisé en ce que** l'unité d'analyse (21) est conçue de telle sorte que la recirculation dans la fistule $R_A$ soit calculée à partir du rapport k du volume sanguin RBV ou de la grandeur corrélée au volume sanguin avant et après la modification du taux de substitution $Q_S$ et du débit $Q_{FM}$ du liquide retiré par la membrane du dialyseur ou filtre et à partir des premier et second coefficients $\alpha$, $\beta$, selon l'équation suivante :

$$R_A = \frac{(1-\alpha)\cdot(1-\beta)\cdot(1-k)}{(1-\alpha)-k\cdot(1-\beta)}\ .$$

9. Dispositif selon l'une quelconque des revendications 1 à 8, **caractérisé en ce que** l'unité d'analyse (21) est conçue de telle sorte que la recirculation cardiopulmonaire $R_{CP}$ soit calculée à partir de la différence de la somme R définie de recirculation dans la fistule $R_A$ et de recirculation cardiopulmonaire $R_{CP}$ et de la recirculation dans la fistule $R_A$ définie.

10. Dispositif de traitement du sang avec un dispositif selon l'une quelconque des revendications 1 à 9 pour la détermination de la fraction de recirculation dans une fistule ($R_A$) et/ou de recirculation cardiopulmonaire ($R_{CP}$) par rapport à la somme de recirculation dans la fistule ($R_A$) et de recirculation cardiopulmonaire ($R_{CP}$).

11. Dispositif de traitement du sang selon la revendication 10, **caractérisé en ce que** le dispositif de traitement du sang présente :

un dialyseur (1) ou filtre qui est subdivisé par une membrane (2) en une première chambre (3) et une seconde chambre (4),
une circulation sanguine extracorporelle (5A) qui présente une conduite d'acheminement de sang (6) conduisant à la première chambre (3) du dialyseur (1) ou filtre et une conduite d'évacuation de sang (7) partant de la première chambre du dialyseur ou filtre,
un système de liquide (5B) qui inclut la seconde chambre (4) du dialyseur (1) ou filtre,
un ensemble de substitution (16) comportant une première conduite de substituant (15, 15a) conduisant à un premier site d'addition (15A) sur la conduite d'acheminement de sang (6) pour l'acheminement de liquide de substitution en amont du dialyseur (1) ou filtre et/ou une seconde conduite de substituant (15, 15b) conduisant à un second site d'addition (15B) sur la conduite d'évacuation de sang (7) pour l'acheminement de liquide de substitution en aval du dialyseur ou filtre,
un ensemble d'ultrafiltration (13, 14) avec lequel du liquide peut être retiré du sang par l'intermédiaire de la membrane (2) du dialyseur (1).

Fig. 1

Fig. 2

Fig. 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0189561 A
- DE 19702441 C1
- DE 19528907 A
- US 6537240 B2
- WO 0145770 A1
- WO 2006072271 A1
- US 4267040 A

**In der Beschreibung aufgeführte Nicht-Patentliteratur**

- **D. SCHNEDITZ et al.** Cardiopulmonary recirculation during hemodialysis. *Kidney Int.,* 1992, vol. 42, 1450-1456
- **W. BAY et al.** Color Doppler flow predicts PTFE graft failure. *J. Am. Soc, Nephrol.,* 1994, vol. 5, 407
- Models to predict recirculation and its effect on treatment time in single-needle dialysis. **F. GOTCH.** First Intl. Symposium on Single-Needle Dialysis. ISAO Press, 1984, 305 ff
- *EDTNA-ERCA Journal,* 1993, vol. 19, 6